# EUROPEAN PATENT SPECIFICATION

(11) **EP 1 337 270 B1**
(45) Date of publication and mention of the grant of the patent: **16.07.2008**
(21) Application number: 01982682.5
(22) Date of filing: 30.10.2001
(51) Int. Cl.: A61K 45/00, A61K 38/21, A61K 31/785, A61P 35/04

(54) **LONG-ACTING INTERFERON AND INTERLUKIN DERIVATIVES AND PHARMACEUTICAL COMPOSITIONS COMPRISING THEM**
INTERFERON- UND INTERLEUKIN-DERIVATE MIT LANGZEITWIRKUNG UND DIESE ENTHALTENDE PHARMAZEUTISCHE ZUSAMMENSETZUNGEN
DERIVES D'INTERFERON ET D'INTERLEUKINE DE LONGUE ACTION ET COMPOSITIONS PHARMACEUTIQUES COMPRENANT CES DERIVES

(30) Priority: 01.11.2000 IL 139400
(43) Date of publication of application: 27.08.2003
(73) Proprietor: YEDA RESEARCH AND DEVELOPMENT CO., LTD., 76100 Rehovot (IL)
(72) Inventor: SHECHTER, Yoram, 76303 Rehovot (IL); FRIDKIN, Matityahu, 72628 Rehovot (IL); GOLDWASER, Itzhak, 75498 Rishon-Le-Zion (IL); PATT, Liana, 69361 Tel Aviv (IL)
(74) Representative: Vossius & Partner
(86) International application number: PCT/IL2001/001005
(87) International publication number: WO 2002/036067

(56) References cited:
- WO-A-98/05361
- SHECHTER, YORAM ET AL: "A new approach for prolonging the half-life of peptides, proteins and low-molecular-weight drugs in vivo" DRUGS OF THE FUTURE, CODEN: DRFUD4, vol. 26, no. 7, 2001, pages 669-676, XP009027170 ISSN: 0377-8282
- MERRIFIELD R B ET AL: "9-(2-Sulfo)fluorenylmethyloxycarbonyl chloride, a new reagent for the purification of synthetic peptides" JOURNAL OF ORGANIC CHEMISTRY, vol. 43, no. 25, 1978, pages 4808-4816, XP002334916 ISSN: 0022-3263
- SHECHTER Y. ET AL.: 'Prolonging the half-life of human interferon-alpha2 in circulation: Design, preparation and analysis of (2-sulfo-9-fluorenylmethoxycarbonyl)7-inter feron-alpha2' PROC. NATL. ACAD. SCI. USA vol. 98, no. 3, 30 January 2000, pages 1212 - 1217, XP002955162
- GERSHONOV ET AL.: 'A novel approach for a water-soluble long-acting insulin prodrug: Design, preparaton and analysis of ((2-sulfo)-9-fluorenylmethoxycarbonyl)3-ins ulin' J. MED. CHEM. vol. 43, 2000, pages 2530 - 2537, XP002955163
- GERSHONOV ET AL.: 'New concept for long-acting insulin, spontaneous conversion of an inactive modified insulin to the active hormone in circulation: 9-fluorenylmethoxycarbonyl derivative of insulin' DIABETES vol. 48, July 1999, pages 1437 - 1442, XP002955164
- BRENNER B.M. ET AL: "Glomerular permselectivity: barrier function based on discrimination of molecular size and charge" AMERICAN JOURNAL OF PHYSIOLOGY, vol. 234, 1978, pages F455-F460,

## Description

The present invention relates to novel interferons and interleukins bearing a functional group sensitive to mild basic conditions, namely 2-sulfo-9-fluorenylmethoxycarbonyl (FMS), and to pharmaceutical compositions comprising them.

**Abbreviations: Fmoc,** 9-fluorenylmethoxycarbonyl; **FMS,** (2-sulfo)-9-fluorenylmethoxycarbonyl; **FMS-OSu,** FMS-N-hydroxysuccinimide; **FMS₃- IL-2,** a human interleukin-2 derivative having three FMS moieties covalently attached to amino groups of IL-2; **FMS₇-IFN-α2,** a human-interferon-α2 derivative having seven **FMS** moieties covalently attached to amino groups of IFN-α2; **HPLC,** high-performance liquid chromatography; **IFN-α2,** Human interferon-α2; **IL,** Interleukin; **IL-2,** Interleukin-2; **PD₅₀,** Protecting dose-50; **RIFS,** reflectometric interference spectroscopy; **SDS-PAGE**, Sodium dodecyl sulphate-polyacrylamide gel electrophoresis; **VSV**, vesicular stomatitis virus.

Cytokines are a large and heterogeneous group of proteins with diverse functions. Some are immunoregulatory proteins synthesized within lymphoreticular cells and play numerous interacting roles in the function of the immune system and in the control of hematopoiesis. In most instances, cytokines mediate their effects via specific cell surface receptors and may act in an autocrine or paracrine manner. Cytokines may also act on or in relevant target cells and appear to act in a manner similar to the mechanism of action of hormones. In other cases, cytokines may have antiproliferative, antimicrobial and antitumor effects.

Cytokines can be divided into several groups, which include interferons (IFNs-α, β and γ), tumor necrosis factor (TNF-α and β), interleukins (IL-1 to IL-18), transforming growth factors (TGF-α and TGF-β), the hematopoietic colony-stimulating factors, e.g. granulocyte colony-stimulating factor (G-CSF), granulocyte-macrophage colony-stimulating factor (GM-CSF), macrophage colony-stimulating factor (M-CSF), erythropoietin (EPO) and thymopoietin (TPO).

The first group of cytokines discovered, the IFNs, are cytokines possessing a variety of antiviral, immunomodulatory and antiproliferative effects. Interferons can be divided into three distinct types (α, β, and γ) associated with specific producer cells and functions. Known collectively as type I interferon, IFNs-α and -β are structurally related and compete for the same cell surface receptor. IFN-γ, also known as type II interferon, is structurally unrelated to type I IFNs, is acid labile, and has a different receptor.

IFN-α is the major IFN produced by leukocytes stimulated by virus, bacteria or protozoa as part of the immune response and can be artificially induced in leukocyte cultures by double-stranded RNA or can be produced by recombinant technology. Multiple subtypes have been identified which contain about 166 amino acids and are variably glycosylated. IFN-α2a and IFN-α2ab are synthetic forms of IFN-α produced by recombinant technology that act as biological response modifiers, and are FDA-approved protein drugs used for the treatment of viral infections including condylomata acuminata, chronic hepatitis C (Barnes *et al.,* 1999), and chronic hepatitis B (Janssen *et al.,* 1999), and as antineoplastic agents in the treatment of Kaposi's sarcoma in HIV-infected patients (Krown, 1991), hairy cell leukemia (Zinzani *et al.,* 1997), and malignant melanoma. IFN-α2 has also shown activity as an anticancer agent in renal cell carcinoma, carcinoid syndrome, and T cell leukemia.

IFN-α2 is administered intramuscularly, subcutaneously or intravenously, with each of these routes giving rise to a different pharmacokinetic pattern. A common feature for any of these administration modes, however, is rapid inactivation of the cytokine in body fluids and in various tissues, leading to the disappearance of IFN-α2 from the plasma within several hours after administration. Unlike many other administered protein drugs, the major route of IFNα2 elimination *in vivo* takes place in the circulatory system through proteolysis and inactivation by serum proteases; only negligible amounts are excreted by the kidneys or removed through receptor mediated endocytosis.

IFN-β is the major interferon produced by fibroblasts in response to stimulation by live or inactivated virus or by certain polynucleotides. It can be produced by cell cultured human fibroblasts or by recombinant technology. Recombinant IFN-β has been approved by the FDA for use in relapsing-type multiple sclerosis.

IFN-γ, also called immune IFN, is produced by immunologically-stimulated lymphocyte cultures. IFN-γ1b, a synthetic form produced by recombinant technology, has been approved for treatment of chronic granulomatous disease.

Interleukin (IL) is a generic term for a group of multifunctional cytokines that are produced by a variety of lymphoid and nonlymphoid cells and have effects at least partly within the lymphopoietic system. At least 18 different interleukins have been decribed to date and they are identified by sequential numbers, e.g. IL-1, IL-2, IL-3 and so on. As the IFNs, also the ILs are produced by recombinant technology.

IL-2 is a natural 15-17 kDa glycoprotein secreted mainly by CD4+ T cells due to antigen activation. It is critical for the development of immune response, inducing proliferation and activation of most of the lymphocytes (T-cells, B-cells and NK cells) after binding to a specific receptor. Notably, IL-2 can activate T lymphocytes and generate activated cytotoxic effectors able to lyse human leukemic blasts, which are resistant to spontaneous natural killer (NK) activity. Recombinant human IL-2 received FDA approval for the treatment of of metastatic renal cell carcinoma and malignant melanoma and orphan drug designation for the treatment of Non-Hodgkin's lymphoma (NHL) and acute myelogenous leukemia (AML). Additional interleukins including IL-1, IL-3, IL-4, IL-6, IL-11 and IL-12 are being clinically investigated.

Tumor necrosis factor (TNF-α and -β) have oncostatic and proinflammatory properties. TNF-α has been extensively tested in the therapy of various malignancies, but results have not been satisfactory. TGF-β is a family of multifunctional cytokines that regulate cellular differentiation, motility and growth and have potential clinical applications in oncology.

The hematopoietic colony-stimulating factors G-CSF, GM-CSF, and M-CSF have been produced by recombinant technology and are being used or investigated for bone marrow transplantation, severe chronic neutropenia, myelodysplasia. Recombinant human erythropoietin (EPO) is in clinical use for treatment of anemia of renal failure, myelodysplasia, and end-stage renal disease.

Protein drugs of molecular weight lower than 50,000 daltons, including most cytokines, are in general short-lived species *in vivo,* having circulatory half-life of about 5-20 min. Clearance of proteins *in vivo* occurs through several mechanisms, including glomerular infiltration in the kidney, receptor-mediated endocytosis and degradation by peripheral tissues, and proteolysis at the tissue surface or by serum proteases. The subcutaneous route of administration provides slower release into the circulation, but there is still a need for technologies that will prolong their half-lives thus enabling the cytokines to exert their beneficial effects for a longer period of time. Considering also that protein drugs are not absorbed orally, prolonged maintenance of therapeutically active drugs in circulation is a desirable feature of primary clinical importance. This condition, however, is rarely achieved following a single administration of low molecular weight peptides and protein drugs.

We have previously described (PCT Publication No. WO 98/05361; Gershonov et al., 2000;) a new conceptual approach for prolonging the half-life of a drug by derivatizing the drug with functional groups sensitive to bases and removable under mild basic, e.g. physiological, conditions. According to this concept, prodrugs are prepared in which at least one amino, hydroxy, mercapto and/or carboxyl groups of the drug molecule are substituted with a radical such as fluorenylmethoxycarbonyl (Fmoc) and 2-sulfo-9-fluorenylmethoxycarbonyl (FMS). Thus, Fmoc and FMS derivatives of peptidic drugs such as insulin and human growth hormone and of non-peptidic drugs such as propanolol, cephalexin , piperacillin, were described.

Prolonging the half-life of protein, particularly cytokine, drugs *in vivo* is a scientific challenge with potentially broad clinical implications. A key question that remained unanswered is whether this approach is also applicable to prolonging half-lives of proteins such as interferons and interleukins which are short-lived *in vivo,* either because of clearance by glomerular infiltration in the kidney or as a result of proteolytic inactivation in the circulatory system and, to a much lesser extent, by receptor-mediated proteolysis.

The present invention thus relates to an interferon (IFN)-α,β or γ or interleukin (IL) in which at least one free amino or hydroxy group is substituted by a radical of the general formula (i): 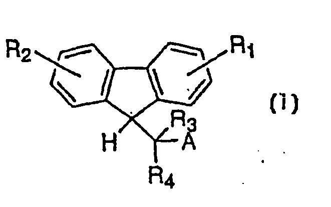 wherein R₁ at position 2 is -SO₃H; R₂, R₃ and R₄ are hydrogen. and A is OCO-, and pharmaceutically acceptable salts thereof.

The cytokine derivative underlying the invention is IFN-α, IFN-β, IFN-γ, or an interleukin (IL) such as any of the known IL-1 to IL-18, or a member of the IL-6 family such as LIF, OSN and CNTF. In preferred embodiments, the cytokine is IFN-α, or IL-2.

The invention further relates to pharmaceutical compositions comprising a cytokine derivative of the invention or a pharmaceutically acceptable salt thereof, and a pharmaceutically acceptable carrier.

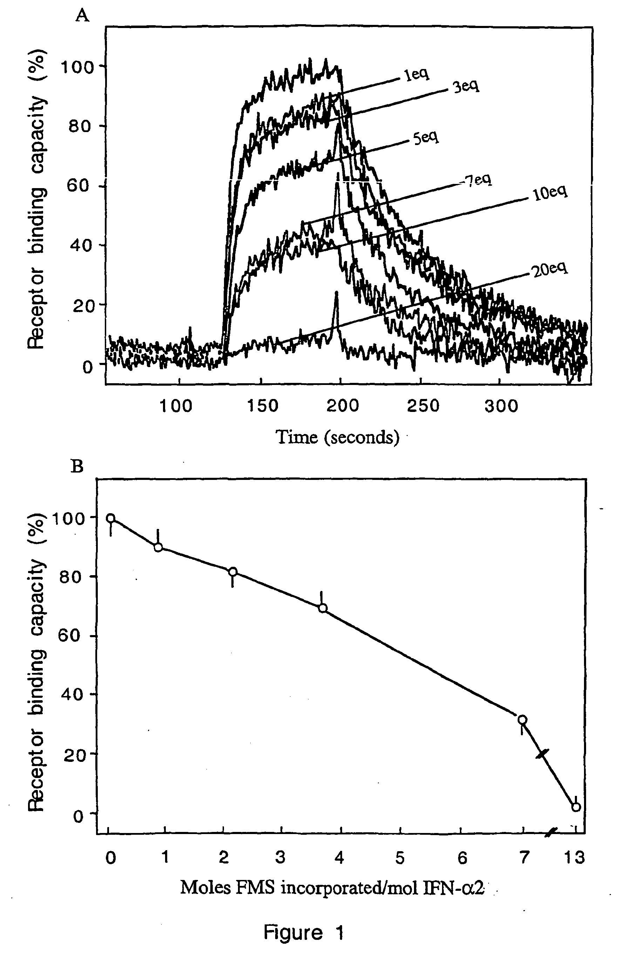

Figs. 1A-1B show progressive modification of the amino acid moieties of EFN-α2 with FMS-OSu and loss of receptor binding capacity as a function of the number of FMS moieties incorporated into IFN-α2. Human IFN-α2 was modified at pH 8.5 with increasing concentrations of FMS-OSu, ranging from 1 equivalent (eq) up to 20 molar equivalents of FMS-OSu. For each treatment, receptor binding capacity (1A) and moles FMS, introduced covalently into IFN-α2 (1B), were determined. Receptor binding capacity toward immobilized extracellular part of IFN-α2 receptor (ifnar2-EC) was assessed by the RIFS procedure. The rising and declining curves represent, respectively, ligand association and ligand dissociation from ifnar2-EC. Moles of FMS/moles of IFNα2 were determined by UV absorption at 301 nm after dialysis and by mass spectroscopy.

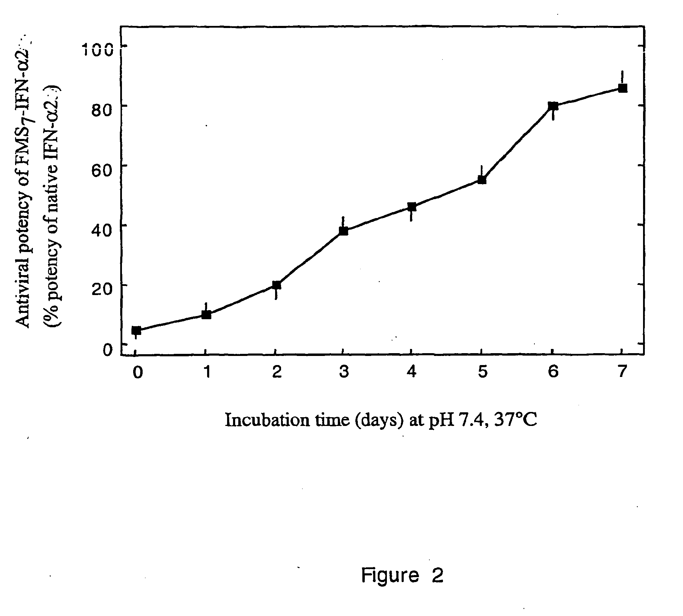

Fig. 2 shows time course of reactivation of FMS₇-IFN-α2 upon incubation at 37°C, pH 7.4. FMS₇-IFN-α2 (1 mg/ml in PBS buffer, pH 7.4) was incubated at 37°C. Aliquots were withdrawn daily and analyzed for their antiviral potency to inhibit VSV-induced cytopathic effects in human WISH cells. Results are expressed as percent of antiviral potency of the native cytokine. Half-maximal inhibition of cytopathic effect was obtained at 0.3 ± 0.03 pM IFN-α2. IFN-α2 derivative exhibiting half-maximal inhibition at a concentration of 30±3 pM was considered as having 1% of the native antiviral potency.

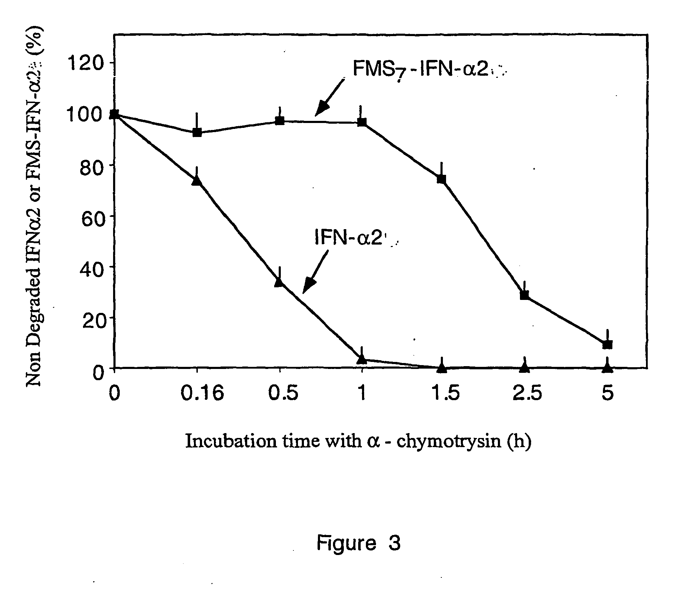

Fig. 3 shows the susceptibility of FMS₇-IFN-α2 toward enzymatic degradation by α-chymotrypsin. FMS₇-IFN-α2 and native IFN-α2 (1 mg/ml each in PBS buffer, pH 7.4) were incubated with α-chymotrypsin (1 % wt/wt). At the indicated time points, aliquots were subjected to analytical HPLC. The quantity of the cytokine (peak area) at t=0 was assigned 100%.

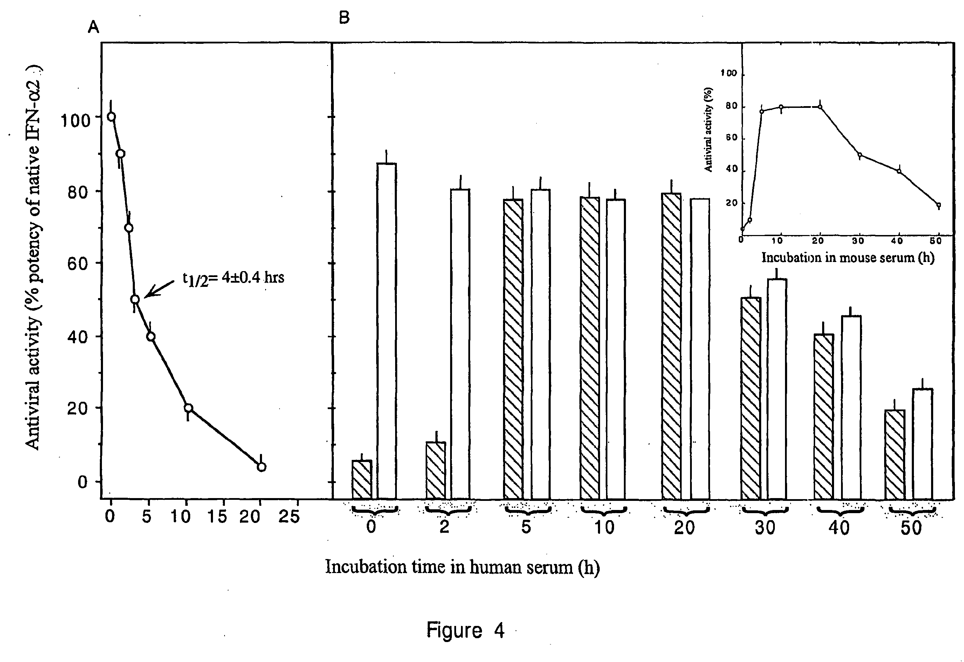

Figs. 4A-4B show the resistance of FMS₇-IFN-α2 to inactivation in human serum. FMS₇-IFN-α2 and native IFN-α2 were incubated in human serum at 37°C. At the indicated time points, aliquots were tested for antiviral potency to inhibit VSV-induced cytopathic effects in human WISH cells. (4A) Antiviral activity of native IFN-α2. (4B) Aliquots of FMS₇-IFN-α2 incubated in serum were either introduced directly to WISH cells (dashed columns) or pre-treated for 18 h at pH 8.5, 37°C (open columns).

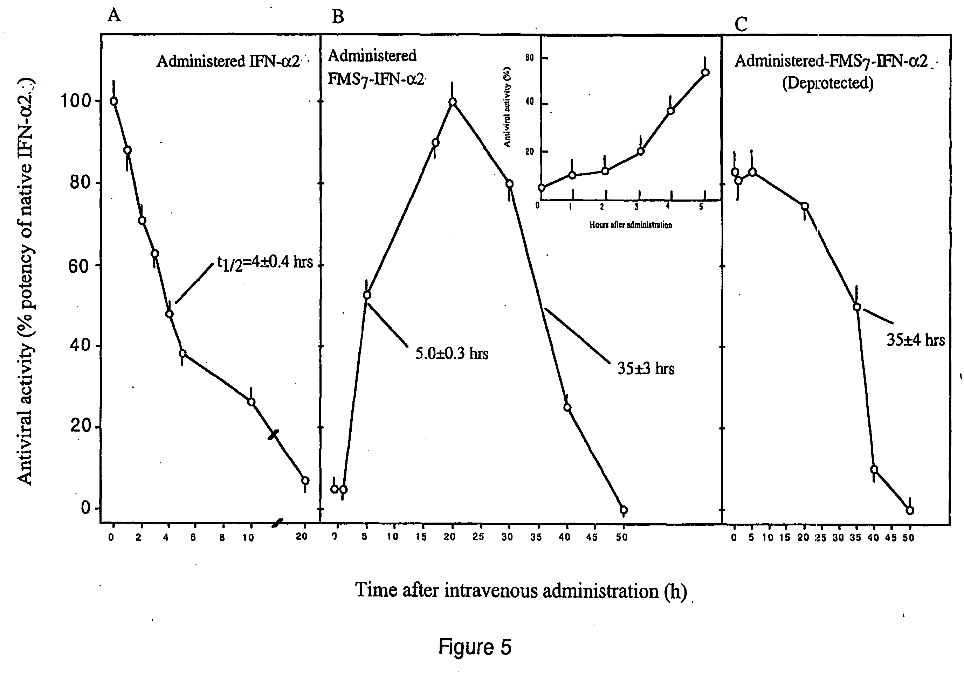

Figs. 5A-5C show that intravenously-administered FMS₇-IFN-α2 in mice facilitates prolonged circulating antiviral activity. Groups of mice (n=5 for each group) received intravenously (10 µg/mouse) native-IFN-α2 (5A) or FMS₇-IFN-α2 (5B and 5C). Blood aliquots were withdrawn at the indicated time points. Circulatory antiviral activities in aliquots were determined in human WISH cells before (5B), and after (5C) an additional period of incubation for 18 h (pH 8.5, 37°C).

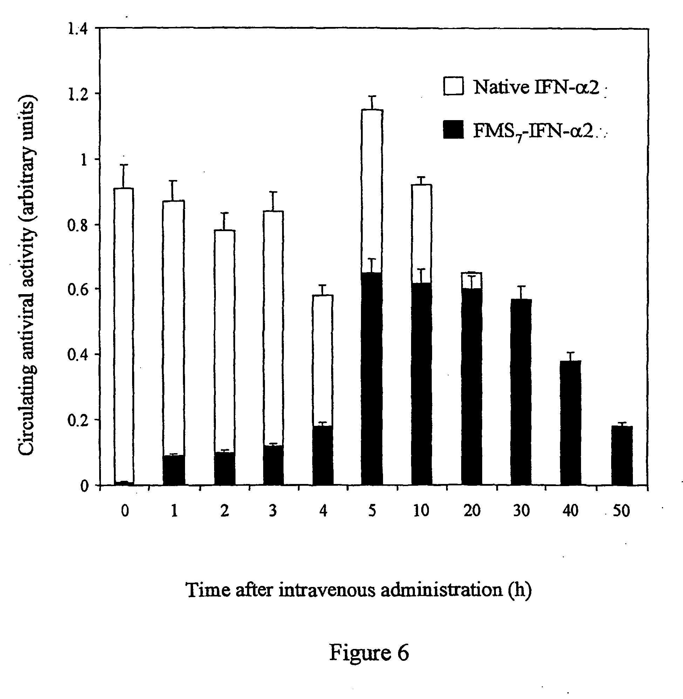

Fig. 6 shows that intravenous coadministration of native IFN-α2 and FMS₇-IFN-α2 facilitates prolonged circulating antiviral activity from the time of administration. Groups of mice (n=3 for each group) received intravenously either native IFN-α2 (10 µg/mouse) or FMS₇-IFN-α2 (10 µg/mouse) or both (10 µg of each). Blood aliquots were withdrawn at the indicated time points and analyzed in human WISH cells for antiviral activity.

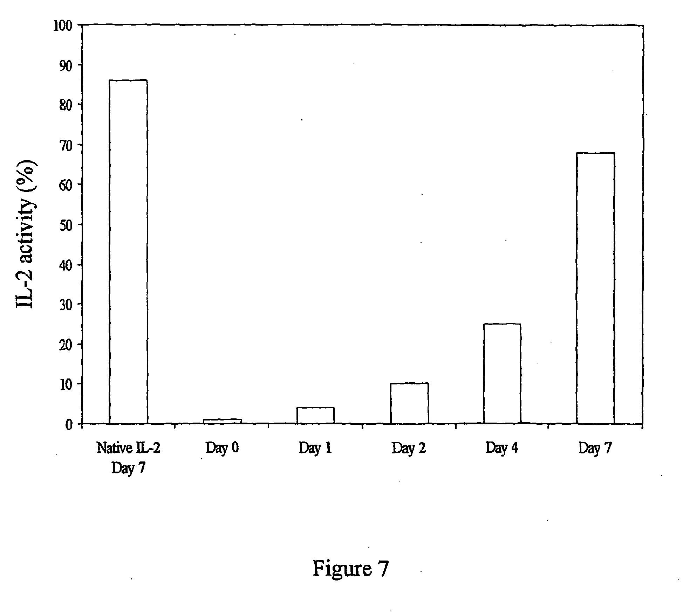

Fig. 7 shows the time course of reactivation of FMS₃-IL-2 upon incubation at 37°C, pH 7.4. FMS₃-IL-2 (1 mg/ml in PBS buffer, pH 7.4) was incubated at 37°C. Aliquots were withdrawn daily and analyzed for their potency to promote proliferative effects in CTL-D cells. Radioactivity due to ³H-thymidine incorporation was measured. High cpm levels correspond to high IL-2 activity.

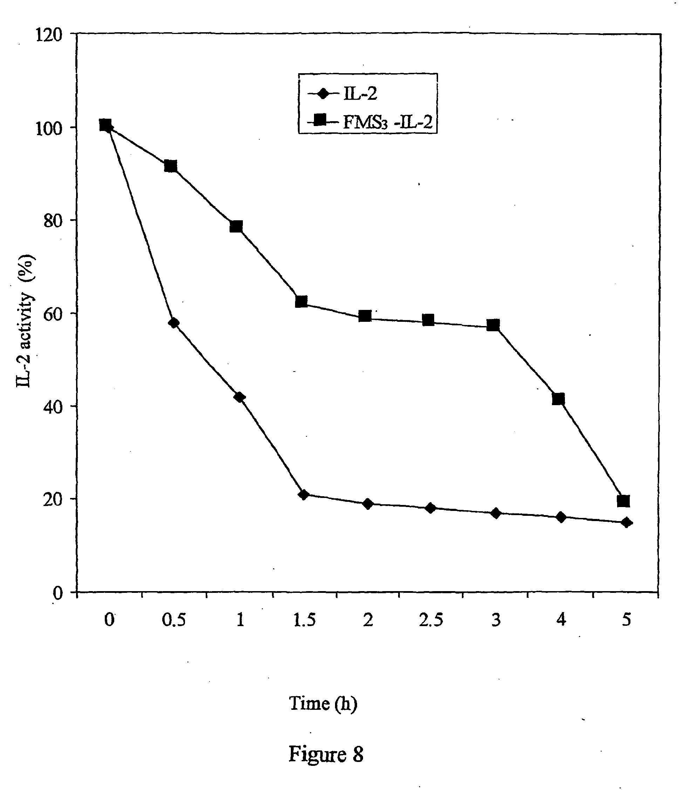

Fig. 8 shows the extended half-life of FMS₃-IL-2 in normal mice circulation. Mice received a single intravenous administration (10 µg) of native IL-2 and FMS₃-IL-2. At the indicated time points, levels of IL-2 were determined by an ELISA procedure.

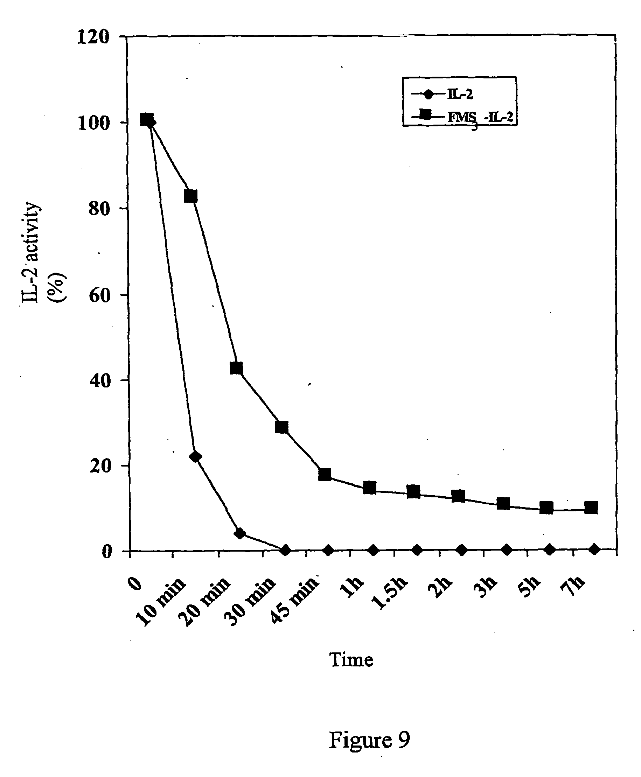

Fig. 9 shows the stability of FMS₃-IL-2 towards enzymatic degradation in comparison to native IL-2. The cytokines were incubated with a mixture of trypsin and chymotrypsin at 37°C. The quantity of the cytokine at each time point was analyzed by HPLC.

According to the present invention, cytokine derivatives (interferons and interleukins) are provided that can be administered as inactive or slightly active prodrugs and are capable of undergoing spontaneous regeneration into the parent bioactive drugs under *in vivo* physiological conditions and in a homogeneous fashion. These cytokine prodrugs of the invention present higher metabolic stability and augmented bioavailability, and represent thus long-lived species as they evade non-specific circulatory and receptor-mediated degradation in the organism.

The slow-releasing cytokine drugs of the present invention can be obtained by derivatization of the original cytokine molecule having free amino or hydroxyl groups, into more hydrophobic derivatives, whereby the native conformation, the biological potency and the recognition identity of the original cytokine by the degradative systems may be lost, rather than preserved, but having the advantage that the thus modified derivatives can slowly and spontaneously hydrolyze back to the native active cytokine drug under the *in vivo* conditions.

The radical (i) that may be covalently linked to at least one free amino or hydroxyl group of the cytokine of the invention, is the (2-sulfo)-9-fluorenylmethoxycarbonyl (FMS) radical, in which A is OCO-.

In the radical (i), R₁ at position 2 is -SO₃H; and R₂, R₃ and R₄, are hydrogen.

Sulfonation of the Fmoc group concomitantly introduces hydrophobic and substantial polar properties and thus the 2-sulfo-Fmoc group is suited for highly hydrophobic proteins such as IFN-α2 and IL-2.

In fact, the above radical (i) belongs to a general family of rare chemical entities that undergo hydrolysis at neutral or slightly alkaline pH and mild conditions, and can therefore be used for temporary reversible protection of α- and ε-amino groups, for example in peptide synthesis, and can be removed from the amino function by a β-elimination reaction, under mild basic conditions.

According to the invention, the radical (i), that is FMS covalently linked to amino and/or hydroxyl moieties, undergoes hydrolysis (via β-elimination) back to the free amino or hydroxy functions, under physiological conditions in the body fluid, namely at pH 7.4 and 37°C.

In one embodiment of the invention, free amino and/or optionally free hydroxyl groups, of the cytokine molecule, are substituted by at least one radical of the formula (i) above.

In a preferred embodiment of the invention, the cytokine derivative has one or more amino groups substituted by the (2-sulfo)-9-fluorenylmethoxycarbonyl (FMS) radical (i), (herein FMS-cytokine).

In one preferred embodiment, the cytokine is an IFN-α, preferably a subtype thereof, most preferably IFN-α2. The preferred derivatives are those in which up to 9 of the total 13 free amino groups of the IFN-α2 molecule are covalently linked to FMS radicals, more preferably 5-7, most preferably, 7 FMS radicals. Thus, the most preferred derivative of IFN-α according to the invention is FMS₇-IFN-α2.

The parent cytokine of the preferred derivative FMS₇-IFN-α2, i.e. IFN-α2, is a recombinant human IFN-α produced in E. coli which has about 165 amino acids and a mol. weight of about 19 kDa. Methods for its preparation and purification have been described (S. Nagata et al., 1980, Nature 284:316; D.W. Goeddel et al., 1980, Nature 287:411). It is clinically used under the names Roferon-A, Canferon, Intron A, and is an FDA-approved drug for the treatment of viral infections including condylomata acuminata, chronic hepatitis B and C, and as antineoplastic agent in the treatment of Kaposi's sarcoma in AIDS patients, hairy cell leukemia, and malignant melanoma. IFN-α2 has also shown activity as an anticancer agent in renal cell carcinoma, carcinoid syndrome, and T cell leukemia. Since FMS₇-IFN-α2 is transformed in the body into the parent cytokine, it is envisaged by the invention that it can be applied for all the clinical uses of the parent cytokine as described above and those uses that may be approved in the future.

As to the mode of administration, FMS₇-IFN-α2 may be administered in the same modes as IFN-α2, i.e. intramuscularly, subcutaneously or intravenously, with each of these routes giving rise to a different pharmacokinetic pattern. However, due to its better stability, FMS₇-IFN-α2 will not undergo rapid inactivation as the parent cytokine in body fluids and in various tissues, leading to the its disappearance from the plasma within several hours after administration. In addition, the modification according to the invention reduces the amount of the derivative to be administered and, therefore, reduces the toxicity presented by the parent cytokine.

In another preferred embodiment, the cytokine is an interleukin, preferably IL-2. IL-2 is known to have a very short half-life (t_{1/2} of 20 min) and to be highly hydrophobic. Thus, in order to prolong its presence in serum, it was chemically modified according to the invention by addition of several FMS groups since sulfonation introduces polar properties.The preferred derivatives are those in which up to 10 of the total 20 free amino groups of the IL-2 molecule are covalently linked to FMS radicals, more preferably 2-5, most preferably, 3 FMS radicals. Thus, the most preferred derivative of IL-2 according to the invention is FMS₃-IL-2.

Human IL-2 is a glycoprotein with a mol weight of about 15 kDa. Methods for the production of recombinant human IL-2 (RhIL-2) have been described (T. Taniguchi et al., 1983, Nature 302:1983; US 4,738,927; EP 142268). RhIL-2 is clinically used under the names Leuferon-2 or Proleukin and is an FDA-approved drug for the treatment of metastatic renal cell carcinoma, malignant melanoma, Non-Hodgkin's lymphoma (NHL) and acute myelogenous leukemia (AML). Since FMS₃-IL-2 is transformed in the body into the parent cytokine, it is envisaged by the invention that it can be applied for all the clinical uses of the parent cytokine IL-2 as described above and those that may be approved in the future. The same advantages explained above for the FMS₇-IFN-α2a derivative are valid for the FMS₃-IL-2 derivative with regard to the parent cytokine.

The cytokine prodrugs of the invention may be prepared by reaction of the cytokine molecule with a suitable reagent comprising a radical (i). Several derivatives of 9-fluorenylmethyl (Fm) are available, such as reference 9-fluorenylmethyl-N-hydroxysuccinimide (Fmoc-OSu) and 2-sulfo-9-fluorenylmethyl-N-hydroxysuccinimide (FMS-OSu), reagents that are very specific for amino functions; reference 9-fluorenylmethoxycarbonyl chloride (Fmoc-Cl) that reacts with, and covalently attaches to, amino and hydroxyl radicals; reference 9-chloromethylfluorene (Fm-Cl) that reacts with mercapto radicals to yield S-Fm derivatives; and reference 9-fluorenylmethanol (Fm-OH) that reacts with, and esterifies, carboxylic functions.

The wide spectrum of chemical procedures available for the production of the cytokine prodrugs of the invention allow to obtain derivatives with a rapid or slow rate of reactivation as necessary. Thus, given cytokine prodrugs may be prepared with physical attributes such as decreased solubility and, therefore, a slower rate of subcutaneous absorption. In addition, altering the hydrophobicity index of cytokine prodrugs, combined with the feature of spontaneous regeneration in blood circulation, may permit to convert orally nonabsorptive cytokine drugs into gastrointestinal permeable cytokine prodrugs.

In another aspect, the present invention relates to pharmaceutical compositions comprising the cytokine derivative of the invention or a pharmaceutically acceptable salt thereof and a pharmaceutically acceptable carrier.

Any suitable route of administration of the cytokine drugs of the invention to humans is envisaged by the invention, for example via conventional injectable, intramuscular, intravenous or subcutaneous administration. These preparations can be prepared by conventional methods known to those skilled in the art, for example as described in "Remington's Pharmaceutical Science", A.R Gennaro, ed., 17th edition, 1985, Mack Publishing Company, Easton, PA, USA.

In another aspect, it is described herein the treatment of a disease or disorder that can be treated with a cytokine drug of the invention which comprises administering to an individual in need thereof a suitable amount of a cytokine derivative of the invention. Any disease or disorder known today or to be discovered in the future that can be treated with a parent cytokine such as, but not limited to, a variety of viral infections, solid and non-solid cancers, bone marrow and organ transplantation, and autoimmune diseases such as multiple sclerosis, is envisaged according to the invention for treatment with the respective derivative of the cytokine according to the present invention.

The invention will now be illustrated by the following Examples.

### EXAMPLES

### Materials and Methods

### (a) Materials

(i) *Non-glycosylated IFN-α2* was prepared as described in detail by Piehler and Schreiber, 1999. Briefly, the gene coding for IFN-α2 was cloned into a two-cistron expression vector to yield the plasmid pT72Cα2. The codons for the first 23 amino acids were changed to improve the level of expression (Schoner *et al.,* 1990). The protein was made in TG1 cells containing the plasmid. IFN-α2 was found in inclusion bodies, which were dissolved in 8 M urea containing 5 mM DTT, and refolded after 20-fold dilution. Purification was conducted by two consecutive procedures of ion-exchange chromatography (Q Sepharose and HiTrap Q, Pharmacia). The IFN-α2a thus prepared runs as a single band of 18 kDa on SDS-PAGE. Concentration was determined by using an extinction coefficient of ε₂₈₀=18,070 (Gill and von Hippel, 1989).
(ii) *Fluorenylmethoxycarbonyl-N-hydroxysuccinimide (Fmoc-OSu)* was purchased from Nova Biochem (Laüfelfingen, Switzerland).
(iii) *FMS-OSu* (2-sulfo-9-fluorenylmethoxycarbonyl-N-hydroxysuccinimide) was prepared essentially by the procedure of Merrifield and Bach, 1978, with slight modifications. Fmoc-OSu (337.4 mg, 1 mmole) was dissolved in 4 ml of dichloromethane and cooled to 0°C. A cold solution of ClSO₃H (60 µl, 0.9 mmole) in 2 ml dichloromethane was added with constant stirring and cooled (0°C) over a period of 15 min. The yellow-turning solution was allowed to warm to room temperature and a white precipitate was formed within one hour. At two hours, cyclohexane (4 ml) was added to dissolve the unreacted Fmoc-OSu. The suspension was centrifuged and the solid material washed 4 times with 6 ml of 1:1 cyclohexane:dichloromethane (vol/vol). The white solid thus formed was dried under P₂O₅ *in vacuo* for 24 h and had the following characteristics: yield 290 mg (86%); mp 140-146°C; TLC (1-butanol/acetic acid/water, 8:1:1) *Rf* 0.31; and mass spectrum (ES⁻) *m*/*z* 416 (100%, M⁻¹). FMS moieties, either free or covalently bound to proteins, absorb at the UV range with Molar Extinction Coefficients ε280 = 21,200 and ε301 = 10,300.
(iv) *Human recombinant Interleukin-2* was purchased from Peprotech Inc., Princeton Business Park, C2, POB 275, Rocky Hill, N.J. 08553, USA.

All other materials used in this study were of analytical grade.

### (b) Methods

(i) *Receptor binding affinities.* The interaction between the recombinant extracellular part of IFN-α2 receptor (ifnar2-EC) and IFN-α2 was monitored under flow-through conditions by an optical probe called reflectometric interference spectroscopy (RIFS) (Striebel et al, 1994). This method detects biomolecular interactions of ligands to transducer-bound proteins as a shift in the interference spectrum caused by change of the apparent optical thickness of the transducer chip and has been described in detail (Piehler and Schreiber, 1999; Striebel *et al.,* 1994; Piehler *et al.,* 1996). A shift of 1 pm corresponds to approximately 1 pg/mm² protein on the surface. The transducer surface was modified with a dextran layer and carboxylated by reaction with molten glutaric anhydride (Sigma) at 75° for 2-8 h. On such surfaces, electrostatic preconcentration and covalent immobilization of proteins were carried out by standard BIAcore protocols. After this procedure, ifnar2-EC was immobilized into a carboxylated dextran layer (Piehler and Schreiber, 1999). All measurements were carried out in 50 mM Hepes (pH 7.4) containing 150 mM NaCl and 0.01% Triton X-100. A sample of 0.8 ml was injected for 80 s with a data acquisition rate of 1 H_{z}. Flow rates of 50 µl/s were applied. Under these conditions, the samples in the flow cell were exchanged within one second, allowing the analysis of processes within 5 seconds.
(ii) *Antiviral activity.* Antiviral activity of IFN-α2 and its derivatives was determined by the capacity of the cytokine to protect human amnion WISH cells against vesicular stomatitis virus (VSV)-induced cytopathic effects (Rubinstein et al., 1981). WISH cells (4.5x10⁵ cells/ml) were seeded in a 96-well plate (100 µl/well) and incubated with two-fold serial dilutions of IFN-α2 or its derivatives for 18 h at 37°C. WISH-cell viability was determined by measuring the absorbance of crystal-violet stained cells in an ELISA plate. In this assay, native IFN-α2 shows 50% protection of VSV-induced WISH cells (ED₅₀) at a concentration of 0.3±0.04 pM. An IFN-α2 derivative exhibiting ED₅₀ of 3±0.3 pM in this assay was considered as having 10% of the native antiviral potency.

### Example 1. Preparation of FMS₇-IFN-α2

FMS₇-IFN-α2 was prepared as follows: To a stirred, cooled (4°C) solution of IFN-α2 [0.95 mg (50 nmol) in 1.0 ml, 0.02 M NaHCO₃], three 5-µl aliquots of a fresh solution of 33 mM FMS-OSu in DMSO were added at 0, 15 and 30 min. Altogether, 10 equivalents of FMS-OSu (500 nmol) over the protein were added. After one hour, the reaction mixture was dyalized overnight at 7°C against H₂O and further purified by reverse-phase HPLC (Spectra-physics SP8800), by using a prepacked column, C₄-Vydac (250 x 6.4 mm), and applying a linear gradient between solutions A (0.1% TFA in H₂O) and B (0.1% TFA in acetonitrile/H₂O, ratio of 75:25). The main peak (emerged with retention time of 37.80 min) was collected and lyophilized. FMS₇-IFN-α2 absorbs at 301 nm with a molar extinction coefficient ε₃₀₁ = 77,000 and has the expected mass for IFN-α2a containing 7±0.2 FMS moieties (Table I).

**Table I Chemical features of FMS₇-IFN-α2**

| Characteristic | | Numerical value |
|---|---|---|
| Moles FMS/mol IFN-α2* | | 7.0±0.2 |
| | Absorbance at 280 nm^{a} | ε 280 =166,500 |
| | Absorbance at 301 nm^{b} | ε 301 = 76,200 |
| | | |
| Mass Spectra^{c} | | |
| | Calculated | 21,383 daltons |
| | Found | 21,382 daltons |
| | | |
| Solubility in aqueous buffer, pH 7.4 | | >0.5 mg/ml |
| | | |
| Retention time (analytical HPLC)^{d} | | 37.80 min |
| | | |
| Reversion to native IFN-α2 upon incubation at pH 8.5, 37°C for the following durations (h)^{e} | | |
| | 4 | 3 |
| | 9 | 10 |
| | 20 | 30 |
| | 50 | 97 |

| | | |
|---|---|---|
| * Determined by UV spectroscopy, measuring the absorbance at 280 and 301 nm. Derivative concentration was determined by acid hydrolysis of a 20-µl aliquot, followed by amino acid analysis; calculated according to aspartic acid (14 residues), alanine (9 residues), and isoleucine (8 residues). (a) Native IFN-α2 absorbs at 280 nm with ε 280 = 18,070. (b) Native IFN-α2 absorbs at 301 nm with ε 301 = 4,100. (c) Mass spectra were determined by using the electrospray ionization technique. (d) IFN-α2 elutes under identical analytic HPLC procedure with retention time = 34±0.2 min. (e) Determined by analytical HPLC procedure (increase in peak area corresponding to native IFN-α2). | | |

### Example 2. Progressive modification of amino acid moieties of IFN-α2 with FMS-OSu.

Proteins can incur a varying degree of covalent modification of 'surface' amino acid moieties before being inactivated, provided that the modification is not directed toward an unusually reactive moiety, often involved in substrate binding and/or in catalysis. Fig. 1A summarizes a set of experiments in which samples of IFN-α2 were modified with increasing concentrations of FMS-OSu. Upon reacting IFN-α2 with 1, 3, 5, 10 and 20 molar excess of FMS-OSu, the respective receptor binding affinities were 90±3; 82±4; 69±3; 33±3 and 3±0.3% of the native cytokine binding potency (Fig. 1A). Measuring UV absorption of the modified cytokine at 301 nm and applying mass spectroscopy, we found that for each such treatment the number of FMS moieties incorporated into IFN-α2 was 0.7, 2.0, 3.5, 7.0, and 10.0 mol FMS/IFN-α2, respectively. Thus, the receptor-binding affinity gradually decreases as a function of the number of FMS moieties incorporated into the cytokine (Fig.1B), although substantially binding affinity remains even when seven mol of FMS are covalently introduced into the protein. Notably, 3 of the 13 amino side-chain moieties of IFN-α2 are not accessible to derivatization by FMS-OSu (20-fold excess, Fig. 1B). The derivative FMS₇-IFN-α2 was selected for further characterization.

### Example 3. Chemical characterization of FMS₇-IFNα2.

Table 1 summarizes the main chemical features of HPLC-purified FMS₇-IFN-α2. This derivative contains 7 mol of FMS/mol protein as deduced by its absorption spectra at 280 nm (ε₂₈₀ = 166,500) and at 301 nm (ε₃₀₁=76,200), values that correspond to the absorption of 7 mol of FMS plus that of the native protein. Mass spectrum analyses revealed the corrected mass of 21,383 daltons, as calculated for IFN-α2 containing seven moieties of FMS (calculated mass = 21,382 daltons, Table 1). The derivative is soluble in aqueous solutions (pH 7.4). It migrates as a single peak on HPLC with a retention time value of 37.80 min. FMS7-IFN-α2 almost fully reverted to the native protein upon incubation at pH 8.5 (37°C) for 2 days, as judged by analytic HPLC procedure (Table 1).

### Example 4. FMS₇-IFN-α2 undergoes hydrolysis at physiological pH and temperature with the generation of the cytokine antiviral potency.

Fig. 2 displays the recovery of the antiviral potency of FMS₇-IFN-α2 following incubation in PBS buffer (pH 7.4) at 37°C. Aliquots were withdrawn daily and antiviral activity was assessed in VSV-induced WISH cells. FMS₇-IFN-α2 has generated antiviral potency in a slow and homogeneous fashion with a t_{1/2} value of 4.0 ± 0.4 days (Fig. 2). After 7 days of incubation, FMS₇-IFN-α2 regained 87 ± 4% of the native cytokine antiviral potency. FMS₁₀-IFN-α2, a derivative in which 10 out of the 13 amino side-chain moieties of IFN-α2 were modified with FMS, has regained only little antiviral potency upon prolonged incubation at physiological pH and temperature, most likely as a result of certain irreversible structural alteration (not shown).

### Example 5. FMS₇-IFN-α2 is resistant to proteolysis.

Fig. 3 describes the treatment of native IFN-α2 and FMS₇-IFN-α2 with 1% α-chymotrypsin in PBS buffer (pH 7.4) at 37°C. Aliquots were withdrawn during proteolysis, then acidified and analyzed by RP-HPLC for evaluating the extent of degradation. Under these conditions, native IFN-α2 and FMS₇-IFN-α2 were proteolyzed by a-chymotrypsin, with a t_{1/2} value of 20 ± 3 min and 120 ± 10 min, respectively. Thus, FMS₇-IFN-α2 is fairly resistant to proteolysis by α-chymotrypsin relative to the native cytokine.

### Example 6. FMS₇-IFN-α2 is resistant to inactivation in human serum.

Native IFN-α2 is a short-lived species *in vivo,* predominantly because of inactivation by serum proteases (see Background section). Hence, the inactivation profile of IFN-α2 incubated in serum *in vitro* at 37°C is expected to be rather similar in many aspects to the pharmacokinetic profile of the native cytokine after intravenous administration in rodents. Fig. 4 shows a set of experiments in which either IFN-α2 or FMS₇-IFN-α2 were incubated in human serum at 37°C for various lengths of time, and their antiviral potencies were determined. With serum-incubated FMS₇-IFN-α2, antiviral activity was determined in samples taken before and after an additional period of incubation for 18 h, at pH 8.5, 37°C, to ensure full FMS hydrolysis.

The antiviral activity of native IFN-α2 declined under these physiological conditions with t_{1/2} = 4.0 ± 0.4 h, reaching undetectable levels 20 h after incubation (Fig. 4A). With FMS₇-IFN-α2, little antiviral activity was found within the first two hours of incubation (Fig. 4B, hatched columns). Activity increased steeply, peaking at 5 h, then maintained at a high plateau over a period of 15 h before declining with t_{1/2} of 25 ± 2h (Fig. 4B, and inset). A high level of antiviral potency was found in the serum aliquot withdrawn at 2 h of incubation after FMS₇-IFN-α2 deprotection (Fig. 4B, open columns).

### Example 7. Intravenously administered FMS₇-IFN-α2 in mice facilitates antiviral activity over prolonged period.

In the experiments summarized in Fig. 5, either IFN-α2 or FMS₇-IFN-α2 was administered intravenously to groups of mice (n=5 in each group). At various time points, blood aliquots were withdrawn and analyzed for their antiviral activity in WISH cells against VSV-induced cytopathic effects. In mice receiving FMS₇-IFN-α2, each blood aliquot was measured for its antiviral activity before (Fig. 5B) and after (Fig. 5C) an additional period of incubation for 18 h at pH 8.4, 37°C, to effect FMS hydrolysis and reactivation of the FMS-modified protein that has not been reactivated *in situ.* After administration of the native cytokine, circulating antiviral activity declined with a t_{1/2} value of 4 ± 0.3 h, reaching undectetable levels at ~20 h after administration (Fig. 5A). Fig. 5B shows the kinetic behavior of intravenously administered FMS₇-IFN-α2 in mice. Only after a lag period of 2 h (Fig. 5B, inset) there is a noticeable elevation in antiviral activity. Circulating antiviral activity was then steeply increased, reaching 50% the native potency at 5 h (Fig. 5B). Antiviral potency is further elevated to maintain a 'wide plateau' between 17 and 30 h after administration, before declining with a t_{1/2} value of 35 ± 3h (Fig. 5B).

Fig. 5C shows the antiviral potencies of the same aliquots afetr hydrolysis of the FMS moieties by an additional incubation period. A high level of antiviral potency was obtained at earlier time points after administration and maintained over a period of 20 h. Antiviral activity was then decreased with a t_{1/2} value of 35 ± 3 h (Fig. 5C). Thus, starting 2 h after intravenous administration of FMS₇-IFN-α2 into mice, there is a prolonged high level of circulating antiviral activity, which lasts over a period of 40 hours.

To examine whether FMS₇-IFN-α2 is antigenic, the cytokine derivative was administered to mice in complete Freund's adjuvant. No antibodies to either FMS₇-IFN-α2 or to the native cytokine could be detected (not shown).

Because circulating antiviral activity in FMS₇-IFN-α2-administered mice emerged after a lag period of 2 hours (Fig. 5B), we next coadministered the native cytokine together with FMS₇-IFN-α2 (10 µg each per mouse), as summarized in Fig. 6. As expected, under these experimental conditions, no lag period was found. A high level of antiviral activity has been initiated shortly after administration and maintained over a prolonged period, before declining with a t_{1/2} value of 37 ± 2 h (Fig. 6).

FMS₇-IFN-α2 administered to mice s.c. or intraperitoneally has maintained prolonged circulating antiviral activities as well, exceding several times that of the native cytokine administered under identical experimental conditions (data not shown).

Summarizing the above experiments: Following the modification of IFN-α2 with increasing concentrations of FMS-OSu, we initially discovered that progressive derivatization is linearly correlated with a decrease in antiviral potency, but as many as 7 FMS-moieties can be introduced into IFN-α2 before the antiviral potency is substantially lost (Figs. 1,2). HPLC-purified FMS₇-IFN-α2 was selected for further studies and chemically characterized. Reversion of FMS₇-IFN-α2 to the native protein upon incubation was confirmed by HPLC (Table I) and by regaining the antiviral potency of FMS₇-IFN-α2 upon incubation at 37°C, pH 7.4 (Fig. 2). We next examined whether FMS₇-IFN-α2 is resistant to general proteolysis using α-chymotrypsin. Derivatization of IFN-α2 with FMS moieties protected significantly the protein against such proteolysis. The antiviral potency of FMS₇-IFN-α2 upon incubating the derivative in human serum at 37°C was then monitored. Under these conditions, antiviral activity appeared at 2 h, peaked at 5 h, and was maintained elevated for a period of 40 h (Fig. 4, inset).

We next compared the pharmacokinetic profile of FMS₇-IFN-α2 versus the native protein following intravenous administration to mice. The native cytokine and its derivative showed a remarkably similar pharmacokinetic profile to the one obtained *in vitro* in human serum (Fig. 4 versus Fig. 5). Thus, as previously suggested, IFN-α2 is a short-lived protein *in vivo* predominantly because of inactivation in the serum. We conclude that our technology is also applicable toward protein drugs that are excluded *in situ* by this mechanism.

In summary, according to the invention, we have linked covalently seven moieties of 9-fluorenylmethoxycarbonyl-SO₃H (FMS) to the amino groups of IFN-α2a. The derivative thus obtained (FMS₇-IFN-α2) has ~4% of the biological potency and 33±4% the receptor binding capacity of the native cytokine. Upon incubation, FMS₇-IFN-α2 undergoes time-dependent spontaneous hydrolysis, generating active interferon with t_{1/2} values of 24±2 h at pH 8.5 and 98±10 h at pH 7.4. When native IFN-α2 is administered to mice intravenously, circulating antiviral activity persists for a short duration and then declines with t_{1/2} = 4 ± 0.5 h, reaching undetectable values - 20 h after administration. With intravenously administered FMS₇-IFN-α2, a lag period of 2 h was observed, followed by a progressive elevation in circulating antiviral-active protein that peaked at 20 h and declined with t_{1/2} = 35 ± 4 h. FMS₇-IFN-α2 is resistant to general proteolysis by α-chymotrypsin and to proteolytic inactivation by human serum proteases *in vitro.*

To sum up, we have introduced here an inactive IFN-α2 derivative, characterized by resistance to *in situ* inactivation and by the capability of slowly reverting to the native active protein at physiological conditions *in vivo* and *in vitro.* Having these attributes, FMS₇-IFN-α2 maintains prolonged circulating antiviral activity in mice, which exceeds 7-8 times the activity obtained after administration of the native cytokine.

### Example 8. Preparation of FMS₃-IL-2

IL-2 is a 15.5 kDa (153 amino acids) glycoprotein containing three cysteines residues, one disulphide bond between the cysteines located at position 58 and 105, and 11 lysine residues to which FMS moieties may be attached. Five equimolar ratios of FMS-OSu molecules were reacted with recombinant human IL-2. Following purification by HPLC, the IL-2 derivative was analyzed by mass and UV spectrometry and HPLC under the same experimental conditions applied to FMS₇-IFN-α2a in Example 1 above. As with FMS₇-IFN-α2, HPLC analysis revealed that FMS₃-IL-2 is fully reverted to the native cytokine upon incubation at pH 8.5 and 37° C for 24 h.

### Example 9. Recovery of biological activity of FMS₃-IL-2

The recovery of the biological activity of FMS₃-IL-2 in comparison to native IL-2 was carried out with a CTL-D cell line as described before (Baker et al., 1979), which proliferation is IL-2 dependent These cells were used as an *in vitro* model for IL-2/FMS₃-IL-2 activity. Following cytokine incubation and ³H-thymidine incorporation, radioactivity was measured by standard methods.

The time course of reactivation of FMS₃-IL-2 was measured upon incubation of the CTL-D cells at 37° C, pH 7.4, with FMS₃-IL-2 (1 mg/ml in PBS buffer, pH 7.4). At different time intervals aliquots were taken for biological activity evaluation by analysis of their potency to promote proliferative effects in CTL-D cells. Radioactivity due to ³H-thymidine incorporation was measured. High cpm levels correspond to high IL-2 activity. The results, depicted in Fig. 7, show that the biological activity of FMS₃-IL-2 is mostly recovered within 7 days. There was a slight loss of activity of FMS₃-IL-2 -IL-2 on day 7 (78% activity compared to the native IL-2 incubated at identical experimental conditions).

### Example 10. Prolonged half-life of FMS₃-IL-2 in normal mice circulation

For pharmacokinetic studies, an *ex-vivo* experiment was carried out in which normal mice (n=3) received a single intravenous administration of 10 µg of native IL-2 or FMS₃-IL-2. At the time points indicated in Fig. 8, serum was withdrawn and was incubated for 24 h at pH 8.5, 37° C, for converting the respective derivative to the native cytokine, and the level of IL-2 was determined by ELISA (Baker *et al.,* 1979) using monoclonal anti-human IL-2 antibodies. In a preliminary assay it was found that FMS₃-IL-2 preserved 83% of the native immunological capacity. The results as summarized in Fig. 8 shows that after administration of native IL-2, a decline of the cytokine levels in sera revealed a t_{1/2} value of ~ 1h, while after administration of FMS₃-IL-2, high circulating levels of IL-2 in serum were obtained with t_{1/2} value of ~ 3h. Therefore, after administration of FMS₃-IL-2, a prolonged duration was shown, as compared to the native cytokine. This is probably attributed to higher resistance of the derivative to enzymatic degradation.

### Example 11. FMS₃-IL-2 is resistant to proteolysis

The susceptibility of FMS₃-IL-2 to proteolysis in comparison to native IL-2 was estimated by incubation with a mixture of trypsin and chymotrypsin (each 0.5%, w/w) at 37° C. Aliquots were withdrawn at various time intervals and the quantity of the cytokine at each time point was analyzed by analytical HPLC. The results are summarized in Fig. 9. Native IL-2 was fully degraded within 30 min, with a t_{1/2} value of 6.6 min. The FMS₃-IL-2 was more resistant to proteolysis, exhibiting a t_{1/2} value of 17.7 min. This stability to proteolysis is probably originated from masking the ε-amino function of lysine, as well as from certain structural alterations and consequent steric hindrance, and therefore reduced binding of the derivatives to the proteases.

### Example 12. Anti-metastatic activity of FMS₃-IL-2

In an *in vivo* experiment designed for the evaluation of the anti-metastatic capacity of FMS₃-IL-2, mice are inoculated intravenously on day (-3) with 10⁵ D122 metastatic cells. Native IL-2 and FMS₃-IL-2 are administered intraperitoneally at high and moderate concentrations (5000 ng and 500 ng, respectively) once daily for 30 days. Each group consists of 8 mice. The original protocol for anti-metastatic therapy implies identical dosages given twice a day. However, since we assume prolongation of FMS₃-IL-2 in serum, it is administered only once a day. Metastatic load in lungs of mice is weighed on day 30.

### References

Baker, P. E., Gillis, S., and Smith, K. A., Monoclonal cytolitic T-cell line, J Exp. Med., 149,273-8 (1979).
Barnes, E., Webster, G., Jacobs, R., and Dusheiko, G., Long-term efficacy of treatment of chronic hepatitis C with alpha interferon or alpha interferon and ribavirin, J Hepatol, 31 Suppl 1, 244-9 (1999).
Gershonov, E., Goldwasser, I., Fridkin, M., and Schechter, Y., A novel approach for a water-soluble long-acting insulin: design, preparation and analysis of [(2-sulfb)-9-fluorenylmethoxycarbonyl)] 3-Insulin, (2000) J Med Chem, in Press *.*
Janssen, H. L., Gerken, G., Carreno, V., Marcellin, P., Naoumov, N. V., Craxi, A., Ring-Larsen, H., Kitis, G., van Hattum, J., de Vries, R. A., Michielsen, P. P., ten Kate, F. J., Hop, W. C., Heijtink, R. A., Honkoop, P., and Schalm, S. W., Interferon alfa for chronic hepatitis B infection: increased efficacy of prolonged treatment. The European Concerted Action on Viral Hepatitis (EUROHEP), Hepatology, 30, 238-43 (1999).
Merrifield, R B., and Bach, A. E., 9-(2-Sulfo)fluorenylmethyloxycarbonyl chloride, a new reagent for the purification of synthetic peptides, J Org Chem, 43, 4808-16 (1978).
Piehler, J., Brecht, A., Geckeler, K. E., and Gauglitz, G., Surface modification for direct immunoprobes, Biosens Bioelectron, 11, 579-90 (1996).
Piehler, J., and Schreiber, G., Biophysical analysis of the interaction of human ifnar2 expressed in E. coli with IFNalpha2, J Mol Biol, 289, 57-67 (1999).
Rubinstein, S., Familletti, P. C., and Pestka, S., Convenient assay for interferons, J Virol, 37, 755-8 (1981).
Schoner, B. E., Belagaje, R M., and Schoner, R. G., Enhanced translational efficiency with two-cistron expression system, Methods Enzymol, 185, 94-103 (1990).
Striebel, C., Brecht, A., and Gauglitz, G., Characterization of biomembranes by spectral ellipsometry, surface plasmon resonance and interferometry with regard to biosensor application, Biosens Bioelectron, 9, 139-46 (1994).
Zinzani, P. L., Lauria, F., Salvucci, M., Rondelli, D., Raspadori, D., Bendandi, M., Magagnoli, M., and Tura, S., Hairy-cell leukemia and alpha-interferon treatment: long-term responders, Haematologica, 82, 152-5 (1997).

## Claims

1. An interferon (IFN)-α, β or γ, or interleukin (IL) in which at least one free amino or hydroxy group is substituted by a radical of the general formula (i): 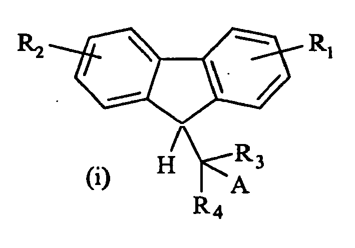 wherein R₁ at position 2 is -SO₃H; R₂, R₃ and R₄ are hydrogen; and A is OCO- (herein FMS-IFN or IL), and pharmaceutically acceptable salts thereof.

2. The IFN of claim 1, wherein said IFN is an IFN-α.

3. The IFN of claim 2, wherein said IFN-α is an IFN-α2.

4. The IFN-α2 of claim 3, in which up to 9 amino groups are substituted with the (2-sulfo)-9-fluorenylmethoxycarbonyl radical (i) wherein R₁ at position 2 is -SO₃H, R₂ to R₄ are hydrogen and A is OCO-.

5. The IFN-α2 of claim 4, in which 7 amino groups are substituted with the (2-sulfo)-9-fluorenylmethoxycarbonyl radical (i) wherein R₁ at position 2 is -SO₃H, R₂ to R₄ are hydrogen and A is OCO- (herein designated FMS₇-IFNα2).

6. The IL of claim 1, wherein said interleukin is IL-2.

7. The IL-2 of claim 6, in which up to 10 amino groups are substituted with the (2-sulfo)-9-fluorenylmethoxycarbonyl radical (i) wherein R₁ at position 2 is -SO₃H, R₂ to R₄ are hydrogen and A is OCO-.

8. The IL-2 of claim 7, in which 3 amino groups are substituted with the (2-sulfo)-9-fluorenylmethoxycarbonyl radical (i) wherein R₁ at position 2 is -SO₃H, R₂ to R₄ are hydrogen and A is OCO- (herein designated FMS₃-IL-2).

9. A pharmaceutical composition comprising an interferon (IFN) or interleukin (IL) according to any one of claims 1 to 8 or a pharmaceutically acceptable salt thereof, and a pharmaceutically acceptable carrier.

## Patentansprüche

1. Interferon (IFN)-α,β oder γ, oder Interleukin (IL), wobei mindestens eine freie Amino- oder Hydroxylgruppe durch einen Rest der allgemeinen Formel (i): 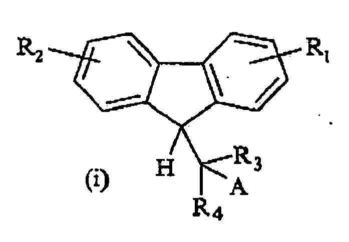 substituiert ist, wobei R₁ auf Position 2 die Bedeutung -SO₃H hat; R₂, R₃ und R₄ ein Wasserstoffatom darstellen; und A die Bedeutung OCO- (hier FMS-IFN oder IL) hat und pharmazeutisch verträgliche Salze davon.

2. IFN nach Anspruch 1, wobei das IFN ein IFN-α ist.

3. IFN nach Anspruch 2, wobei das IFN-α ein IFN-α2 ist.

4. IFN-α2 nach Anspruch 3, wobei bis zu 9 Aminogruppen mit dem (2-Sulfo)-9-fluorenylmethoxycarbonylrest (i) substituiert sind, wobei R₁ auf Position 2 die Bedeutung -SO₃H hat, R₂ bis R₄ ein Wasserstoffatom darstellen und A die Bedeutung OCO- hat.

5. IFN-α2 nach Anspruch 4, wobei 7 Aminogruppen mit dem (2-Sulfo)-9-fluorenylmethoxycarbonylrest (i) substituiert sind, wobei R₁ auf Position 2 die Bedeutung -SO₃H hat, R₂ bis R₄ ein Wasserstoffatom darstellen und A die Bedeutung OCO- (hier als FMS₇-IFNα2 bezeichnet) hat.

6. IL nach Anspruch 1, wobei das Interleukin IL-2 ist.

7. IL-2 nach Anspruch 6, wobei bis zu 10 Aminogruppen mit dem (2-Sulfo)-9-fluorenylmethoxycarbonylrest (i) substituiert sind, wobei R₁ auf Position 2 die Bedeutung -SO₃H hat, R₂ bis R₄ ein Wasserstoffatom darstellen und A die Bedeutung OCO- hat.

8. IL-2 nach Anspruch 7, wobei 3 Aminogruppen mit dem (2-Sulfo)-9-fluorenylmethoxycarbonylrest (i) substituiert sind, wobei R₁ auf Position 2 die Bedeutung -SO₃H hat, R₂ bis R₄ ein Wasserstoffatom darstellen und A die Bedeutung OCO- (hier als FMS₃-IL-2 bezeichnet) hat.

9. Arzneimittel, umfassend ein Interferon (IFN) oder Interleukin (IL) nach einem der Ansprüche 1 bis 8, oder ein pharmazeutisch verträgliches Salz davon, und einen pharmazeutisch verträglichen Träger.

## Revendications

1. Interféron (IFN)-α, β ou γ, ou interleukine (IL) où au moins un groupe amino ou hydroxy libre est substitué par un radical de formule générale (i) : 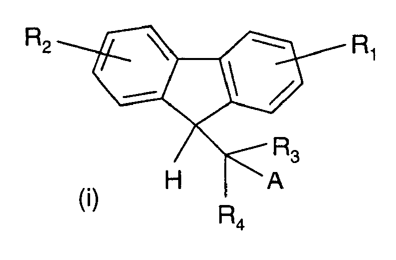 où R₁ à la position 2 est -SO₃H ; R₂, R₃ et R₄ sont l'hydrogène ; et A et OCO- (ici FMS-IFN ou IL), et ses sels pharmaceutiquement acceptables.

2. IFN selon la revendication 1 où ledit IFN est un IFN-α.

3. IFN selon la revendication 2 où ledit IFN-α est un IFN-α2.

4. IFN-α2 selon la revendication 3 où jusqu'à 9 groupes amino sont substitués avec le radical (2-sulfo)-9-fluorénylméthoxycarbonyle (i) où R₁ à la position 2 est -SO₃H, R₂ à R₄ sont l'hydrogène et A est OCO-.

5. IFN-α2 selon la revendication 4 où 7 groupes amino sont substitués avec le radical (2-sulfo)-9-fluorénylméthoxycarbonyle (i) où R₁ à la position 2 est -SO₃H, R₂ à R₄ sont l'hydrogène et A est OCO- (appelé ici FMS₇-IFN-α2).

6. IL selon la revendication 1 où ladite interleukine est IL-2.

7. IL-2 selon la revendication 6 où jusqu'à 10 groupes amino sont substitués avec le radical (2-sulfo)-9-fluorénylméthoxycarbonyle (i) où R₁ à la position 2 est -SO₃H, R₂ à R₄ sont l'hydrogène et A est OCO-.

8. IL-2 selon la revendication 7 où 3 groupes amino sont substitués avec le radical (2-sulfo)-9-fluorénylméthoxycarbonyle (i) où R₁ à la position 2 est -SO₃H, R₂ à R₄ sont l'hydrogène et A est OCO- (appelé ici FMS₃-IL-2).

9. Composition pharmaceutique comprenant un interféron (IFN) ou une interleukine (IL) selon l'une quelconque des revendications 1 à 8 ou un sel pharmaceutiquement acceptable de celui-ci ou celle-ci, et un vecteur pharmaceutiquement acceptable.
